# EUROPEAN PATENT SPECIFICATION

(11) **EP 1 645 640 B1**
(45) Date of publication and mention of the grant of the patent: **21.08.2013**
(21) Application number: 05256228.7
(22) Date of filing: 05.10.2005
(51) Int. Cl.: C12Q 1/68

(54) **Method for detecting chromosomal translocations**
Verfahren zur Detektion von chromosomalen Translokationen
Procédé pour la détection des translocations chromosomales

(30) Priority: 05.10.2004 US 616273 P
(43) Date of publication of application: 12.04.2006
(73) Proprietor: Affymetrix, Inc., Santa Clara, CA 95051 (US)
(72) Inventor: Jones, Keith, W., Sunnyvale, CA 94087 (US); Shapero, Michael, H., Redwood City, CA 94061 (US)
(74) Representative: Bizley, Richard Edward

(56) References cited:
- WO-A2-03/033724
- US-A1- 2003 108 900
- DATABASE BIOSIS [Online] BIOSCIENCES INFORMATION SERVICE, PHILADELPHIA, PA, US; March 2003 (2003-03), DHULIPALA PRASAD D K ET AL: "GenomiPhi amplification of human genomic DNA: utility of amplified DNA in genetic variation experiments." XP9127495 Database accession no. PREV200300391701 & FASEB JOURNAL, vol. 17, no. 4-5, March 2003 (2003-03), pages Abstract No. 371.3 URL-http://ww, FASEB MEETING ON EXPERIMENTAL BIOLOGY: TRANSLATING THE GENOME; SAN DIEGO, CA, USA; APRIL 11-15, 2003 ISSN: 0892-6638
- SHI MICHAEL M: "Enabling large-scale pharmacogenetic studies by high-throughput mutation detection and genotyping technologies" CLINICAL CHEMISTRY, vol. 47, no. 2, February 2000 (2000-02), pages 164-172, XP002561533 ISSN: 0009-9147
- TZVETKOV MLADEN V ET AL: "Genome-wide single-nucleotide polymorphism arrays demonstrate high fidelity of multiple displacement-based whole-genome amplification" ELECTROPHORESIS, WILEY INTERSCIENCE, DE, vol. 26, no. 3, 1 February 2005 (2005-02-01), pages 710-715, XP002371239 ISSN: 0173-0835
- GUNDERSON KEVIN L ET AL: "A genome-wide scalable SNP genotyping assay using microarray technology" NATURE GENETICS, vol. 37, no. 5, May 2005 (2005-05), pages 549-554, XP002561532 ISSN: 1061-4036

## Description

### FIELD OF THE INVENTION

The methods of the invention relate generally to the fields of analysis of genomic DNA.

### BACKGROUND OF THE INVENTION

Single nucleotide polymorphisms (SNPs) have emerged as the marker of choice for genome wide association studies and genetic linkage studies. Building SNP maps of the genome will provide the framework for new studies to identify the underlying genetic basis of complex diseases such as cancer, mental illness and diabetes. Due to the wide ranging applications of SNPs there is still a need for the development of robust, flexible, cost-effective technology platforms that allow for scoring genotypes in large numbers of samples.

US 2003/108900 discloses a multiplex method for amplifying genomic DNA, labelling the amplified DNA and detecting the DNA on an array containing allele-specific probes.

The PCR primers disclosed may be designed to amplify different genomic markers such as translocations or other chromosomal abnormalities. The arrays disclosed include oligonucleotide arrays that incorporate a few to millions of probes.

### SUMMARY OF THE INVENTION

The present invention is defined by appended claims 1-18.

Disclosed are novel methods of sample preparation and analysis comprising managing or reducing the complexity of a nucleic acid sample by amplification of a collection of target sequences using labeled nucleotides and a DNA polymerase with a high processivity rate. The nucleotides may be biotinylated. The amplified collection of target sequences may be analyzed by hybridization to an array that is designed to interrogate sequence variation in the target sequences.

Disclosed is that a sample is enriched for a predetermined subset of the genome by targeting selected regions for amplification using a collection of target specific primers. The regions are initially amplified by extension of the target specific primers using a DNA polymerase capable of extension over more than 5, 10, 15, 40, 50 or 100 kb. Second amplification steps using random amplification methods, such as random priming may also be used to increase the amount of the enriched targets. The resulting amplified and enriched sample is enriched for a subset of sequences in the human genome so that more than 70, 80, 90 or 95% of the sample consists of less than 0.1%, 1% or 5% of the sequences present in the human genome.

A method of genotyping one or more polymorphic locations, in a sample is disclosed. An amplified collection of labeled target sequences from the sample is prepared and hybridized to an array designed to interrogate at least one polymorphic location in the collection of target sequences. The hybridization pattern is analyzed to determine the identity of the allele or alleles present at one or more polymorphic location in the collection of target sequences. The label may be biotin, which can be detected using an anti-streptavidin antibody or the label may be digoxigenin.

Further, a method for analyzing sequence variations in a population of individuals is disclosed. A nucleic acid sample is obtained from each individual and a collection of target sequences from each nucleic acid sample is amplified and labeled. Each labeled amplified collection of target sequences is hybridized to an array designed to interrogate sequence variation in the collection of target sequences to generate a hybridization pattern for each sample and the hybridization patterns are analyzed or compared to determine the presence or absence of sequence variation in the population of individuals.

Fragmentation of the target sequences may occur by digestion with one or more restriction enzymes.

One of the common sequence primers may be resistant to nuclease digestion and the sample is treated with a nuclease that cleaves 5' to 3' after the fragments are extended in the presence of labeled ddNTP. The primer may be resistant to nuclease digestion because it contains phosphorothioate linkages. The nuclease may be T7 Gene 6 Exonuclease.

A method for screening for sequence variations in a population of individuals is disclosed. A nucleic acid sample from each individual is provided and the sample is amplified and genotyped and the genotypes from the samples are compared to determine the presence or absence of sequence variation in the population of individuals.

A plurality of oligonucleotides attached to a solid support is disclosed. The solid support may be arrays, beads, microparticles, microtitre dishes or gels. The oligonucleotides may be released and used for a variety of analysis. The plurality of oligonucleotides may comprise a collection of capture probes.

### BRIEF DESCRIPTION OF THE FIGURES

Figure 1 shows a schematic of method of detecting translocations and mapping breakpoint of translocations.
Figure 2 shows extension of a 5' end labeled primer followed by separation of the unextended primers from the extension reaction by size exclusion chromatography.

### DETAILED DESCRIPTION OF THE INVENTION

### a) General

The present invention has many preferred embodiments and relies on many patents, applications and other references for details known to those of the art.

As used in this application, the singular form "a," "an," and "the" include plural references unless the context clearly dictates otherwise. For example, the term "an agent" includes a plurality of agents, including mixtures thereof.

An individual is not limited to a human being but may also be other organisms including but not limited to mammals, plants, bacteria, or cells derived from any of the above.

Throughout this disclosure, various aspects of this invention can be presented in a range format. It should be understood that the description in range format is merely for convenience and brevity. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example herein below. However, other equivalent conventional procedures can, of course, also be used. Such conventional techniques and descriptions can be found in standard laboratory manuals such as Genome Analysis: A Laboratory Manual Series (Vols. I-IV*),* Using Antibodies: A Laboratory Manual*,* Cells: A Laboratory Manual*,* PCR Primer: A Laboratory Manual*,* and Molecular Cloning: A Laboratory Manual (all from Cold Spring Harbor Laboratory Press), Stryer, L. (1995) Biochemistry (4th Ed.) Freeman, New York, Gait, "Oligonucleotide Synthesis: A Practical Approach" 1984, IRL Press, Lond*on,* Nelson and Cox (2000), Lehninger, Principles of Biochemistry 3rd Ed., W.H. Freeman Pub., New York, NY and Berg et al. (2002) Biochemistry, 5th Ed., W.H. Freeman Pub., New York.

The present invention employs arrays Methods and techniques applicable to polymer (including protein) array synthesis have been described in WO 00/58516, U.S. Patent Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846 and 6,428,752, International Publication No. WO 99/36760 and International Publication No. WO 01/58593.

Patents that describe synthesis techniques in specific embodiments include U.S. Patent Nos. 5,412,087, 6,147,205, 6,262,216, 6,310,189, 5,889,165, and 5,959,098. Nucleic acid arrays are described in many of the above patents, but the same techniques are applied to polypeptide arrays.

Nucleic acid arrays that are useful in the present invention include those that are commercially available from Affymetrix (Santa Clara, CA) under the brand name GeneChip®. Example arrays are shown on the website at affymetrix.com.

Contemplated are many uses for polymers attached to solid substrates. These uses include gene expression monitoring, profiling, library screening, genotyping and diagnostics. Gene expression monitoring and profiling methods can be shown in U.S. Patent Nos. 5,800,992, 6,013,449, 6,020,135, 6,033,860, 6,040,138, 6,177,248 and 6,309,822. Genotyping and uses therefore are shown in U.S. Serial Nos. U.S. Patent Application Publication 20030036069 and US 2004/067493, and U.S. Patent Nos. 5,856,092, 6,300,063, 5,858,659, 6,284,460, 6,361,947, 6,368,799 and 6,333,179. Other uses are embodied in U.S. Patent Nos. 5,871,928, 5,902,723, 6,045,996, 5,541,061, and 6,197,506.

Sample preparation methods may be used in the methods of the invention. Prior to or concurrent with genotyping, the genomic sample may be amplified by a variety of mechanisms, some of which may employ PCR. *See,* for example, PCR Technology: Principles and Applications for DNA Amplification (Ed. H.A. Erlich, Freeman Press, NY, NY, 1992); PCR Protocols: A Guide to Methods and Applications (Eds. Innis, et al., Academic Press, San Diego, CA, 1990); Mattila et al., Nucleic Acids Res. 19, 4967 (1991); Eckert et al., PCR Methods and Applications 1, 17 (1991); PCR (Eds. McPherson et al., IRL Press, Oxford); and U.S. Patent Nos. 4,683,202, 4,683,195, 4,800,159 4,965,188,and 5,333,675. The sample may be amplified on the array. See, for example, U.S. Patent No. 6,300,070 .

Other suitable amplification methods include the ligase chain reaction (LCR) *(for example,* Wu and Wallace, Genomics 4, 560 (1989), Landegren et al., Science 241, 1077 (1988) and Barringer et al. Gene 89:117 (1990)), transcription amplification (Kwoh et al., Proc. Natl. Acad. Sci. USA 86, 1173 (1989) and W088/10315), self-sustained sequence replication (Guatelli et al., Proc. Nat. Acad. Sci. USA, 87, 1874 (1990) and WO90/06995), selective amplification of target polynucleotide sequences (U.S. Patent No. 6,410,276), consensus sequence primed polymerase chain reaction (CP-PCR) (U.S. Patent No. 4,437,975), arbitrarily primed polymerase chain reaction (AP-PCR) (U.S. Patent Nos. 5, 413,909, 5,861,245) and nucleic acid based sequence amplification (NABSA). *(See,* U.S. Patent Nos. 5,409,818, 5,554,517, and 6,063,603). Other amplification methods that may be used are described in, U.S. Patent Nos. 5,242,794, 5,494,810, 4,988,617 and 6,582,938.

Additional methods of sample preparation and techniques for reducing the complexity of a nucleic sample are described in Dong et al., Genome Research 11, 1418 (2001), in U.S. Patent Number 6,361,947, 6,391,592 and U.S. Patent Application Publications 2003096235, 2003082543, and 2003/036069.

Methods for conducting polynucleotide hybridization assays have been well developed in the art. Hybridization assay procedures and conditions will vary depending on the application and are selected in accordance with the general binding methods known including those referred to in: Maniatis et al. Molecular Cloning: A Laboratory Manual (2nd Ed. Cold Spring Harbor, N.Y, 1989); Berger and Kimmel Methods in Enzymology, Vol. 152, Guide to Molecular Cloning Techniques (Academic Press, Inc., San Diego, CA, 1987); Young and Davism, P.N.A.S, 80: 1194 (1983). Methods and apparatus for carrying out repeated and controlled hybridization reactions have been described in U.S. Patent Nos. 5,871,928, 5,874,219, 6,045,996 and 6,386,749, 6,391,623

Disclosed is the use of signal detection of hybridization between ligands in certain preferred embodiments. See U.S. Patent Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in U.S. Application Publication 2004/012676 and in PCT Application WO99/47964.

Methods and apparatus for signal detection and processing of intensity data are disclosed in, for example, U.S. Patents Nos. 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in U.S. Application Publications 2004/012676 and 2005/059062 and in PCT Application WO99/47964.

The practice of the disclosed methods may also employ conventional biology methods, software and systems. Computer software products typically include computer readable medium having computer-executable instructions for performing the logic steps of the method of the invention. Suitable computer readable medium include floppy disk, CD-ROM/DVD/DVD-ROM, hard-disk drive, flash memory, ROM/RAM, magnetic tapes and etc. The computer executable instructions may be written in a suitable computer language or combination of several languages. Basic computational biology methods are described in, for example Setubal and Meidanis et al., Introduction to Computational Biology Methods (PWS Publishing Company, Boston, 1997); Salzberg, Searles, Kasif, (Ed.), Computational Methods in Molecular Biology, (Elsevier, Amsterdam, 1998); Rashidi and Buehler, Bioinformatics Basics: Application in Biological Science and Medicine (CRC Press, London, 2000) and Ouelette and Bzevanis Bioinformatics: A Practical Guide for Analysis of Gene and Proteins (Wiley & Sons, Inc., 2nd ed., 2001). See U.S. Patent Number 6,420,108.

There are disclosed herein various computer program products and software for a variety of purposes, such as probe design, management of data, analysis, and instrument operation. See, U.S. Patent Nos. 5,593,839, 5,795,716, 5,733,729, 5,974,164, 6,066,454, 6,090,555, 6,185,561, 6,188,783, 6,223,127, 6,229,911 and 6,308,170.

There are also disclosed herein methods for providing genetic information over networks such as the Internet as shown in (United States Publication Number 2002183936.

### b) Definitions

The term "admixture" refers to the phenomenon of gene flow between populations resulting from migration. Admixture can create linkage disequilibrium (LD).

The term "allele' as used herein is any one of a number of alternative forms a given locus (position) on a chromosome. An allele may be used to indicate one form of a polymorphism, for example, a biallelic SNP may have possible alleles A and B. An allele may also be used to indicate a particular combination of alleles of two or more SNPs in a given gene or chromosomal segment. The frequency of an allele in a population is the number of times that specific allele appears divided by the total number of alleles of that locus.

The term "array" as used herein refers to an intentionally created collection of molecules which can be prepared either synthetically or biosynthetically. The molecules in the array can be identical or different from each other. The array can assume a variety of formats, *for example,* libraries of soluble molecules; libraries of compounds tethered to resin beads, silica chips, or other solid supports.

The term "biomonomer" as used herein refers to a single unit of biopolymer, which can be linked with the same or other biomonomers to form a biopolymer (for example, a single amino acid or nucleotide with two linking groups one or both of which may have removable protecting groups) or a single unit which is not part of a biopolymer. Thus, for example, a nucleotide is a biomonomer within an oligonucleotide biopolymer, and an amino acid is a biomonomer within a protein or peptide biopolymer; avidin, biotin, antibodies, antibody fragments, etc., for example, are also biomonomers.

The term "biopolymer" or sometimes refer by "biological polymer" as used herein is intended to mean repeating units of biological or chemical moieties. Representative biopolymers include, but are not limited to, nucleic acids, oligonucleotides, amino acids, proteins, peptides, hormones, oligosaccharides, lipids, glycolipids, lipopolysaccharides, phospholipids, synthetic analogues of the foregoing, including, but not limited to, inverted nucleotides, peptide nucleic acids, Meta-DNA, and combinations of the above.

The term "biopolymer synthesis" as used herein is intended to encompass the synthetic production, both organic and inorganic, of a biopolymer. Related to a bioploymer is a "biomonomer".

The term "combinatorial synthesis strategy" as used herein refers to a combinatorial synthesis strategy is an ordered strategy for parallel synthesis of diverse polymer sequences by sequential addition of reagents which may be represented by a reactant matrix and a switch matrix, the product of which is a product matrix. A reactant matrix is a 1 column by m row matrix of the building blocks to be added. The switch matrix is all or a subset of the binary numbers, preferably ordered, between 1 and m arranged in columns. A "binary strategy" is one in which at least two successive steps illuminate a portion, often half, of a region of interest on the substrate. In a binary synthesis strategy, all possible compounds which can be formed from an ordered set of reactants are formed. In most preferred embodiments, binary synthesis refers to a synthesis strategy which also factors a previous addition step. For example, a strategy in which a switch matrix for a masking strategy halves regions that were previously illuminated, illuminating about half of the previously illuminated region and protecting the remaining half (while also protecting about half of previously protected regions and illuminating about half of previously protected regions). It will be recognized that binary rounds may be interspersed with non-binary rounds and that only a portion of a substrate may be subjected to a binary scheme. A combinatorial "masking" strategy is a synthesis which uses light or other spatially selective deprotecting or activating agents to remove protecting groups from materials for addition of other materials such as amino acids.

The term "complementary" as used herein refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%. Alternatively, complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary. See, M. Kanehisa Nucleic Acids Res. 12:203 (1984).

The term "effective amount" as used herein refers to an amount sufficient to induce a desired result.

The term "genome" as used herein is all the genetic material in the chromosomes of an organism. DNA derived from the genetic material in the chromosomes of a particular organism is genomic DNA. A genomic library is a collection of clones made from a set of randomly generated overlapping DNA fragments representing the entire genome of an organism.

The term "genotype" as used herein refers to the genetic information an individual carries at one or more positions in the genome. A genotype may refer to the information present at a single polymorphism, for example, a single SNP. For example, if a SNP is biallelic and can be either an A or a C then if an individual is homozygous for A at that position the genotype of the SNP is homozygous A or AA. Genotype may also refer to the information present at a plurality of polymorphic positions.

The term "Hardy-Weinberg equilibrium" (HWE) as used herein refers to the principle that an allele that when homozygous leads to a disorder that prevents the individual from reproducing does not disappear from the population but remains present in a population in the undetectable heterozygous state at a constant allele frequency.

The term "hybridization" as used herein refers to the process in which two single-stranded polynucleotides bind non-covalently to form a stable double-stranded polynucleotide; triple-stranded hybridization is also theoretically possible. The resulting (usually) double-stranded polynucleotide is a "hybrid." The proportion of the population of polynucleotides that forms stable hybrids is referred to herein as the "degree of hybridization." Hybridizations are usually performed under stringent conditions, for example, at a salt concentration of no more than about 1 M and a temperature of at least 25°C. For example, conditions of 5X SSPE (750 mM NaCl, 50 mM NaPhosphate, 5 mM EDTA, pH 7.4) and a temperature of 25-30°C are suitable for allele-specific probe hybridizations or conditions of 100 mM MES, 1 M [Na⁺], 20 mM EDTA, 0.01% Tween-20 and a temperature of 30-50°C, preferably at about 45-50°C. Hybridizations may be performed in the presence of agents such as herring sperm DNA at about 0.1 mg/ml, acetylated BSA at about 0.5 mg/ml. As other factors may affect the stringency of hybridization, including base composition and length of the complementary strands, presence of organic solvents and extent of base mismatching, the combination of parameters is more important than the absolute measure of any one alone. Hybridization conditions suitable for microarrays are described in the Gene Expression Technical Manual, 2004 and the GeneChip Mapping Assay Manual, 2004.

The term "hybridization probes" as used herein are oligonucleotides capable of binding in a base-specific manner to a complementary strand of nucleic acid. Such probes include peptide nucleic acids, as described in Nielsen et al., Science 254, 1497-1500 (1991), LNAs, as described in Koshkin et al. Tetrahedron 54:3607-3630, 1998, and US Patent No. 6,268,490 and other nucleic acid analogs and nucleic acid mimetics.

The term "hybridizing specifically to" as used herein refers to the binding, duplexing, or hybridizing of a molecule only to a particular nucleotide sequence or sequences under stringent conditions when that sequence is present in a complex mixture *(for example*, total cellular) DNA or RNA.

The term "initiation biomonomer" or "initiator biomonomer" as used herein is meant to indicate the first biomonomer which is covalently attached via reactive nucleophiles to the surface of the polymer, or the first biomonomer which is attached to a linker or spacer arm attached to the polymer, the linker or spacer arm being attached to the polymer via reactive nucleophiles.

The term "isolated nucleic acid" as used herein mean an object species invention that is the predominant species present (i.e., on a molar basis it is more abundant than any other individual species in the composition). Preferably, an isolated nucleic acid comprises at least about 50, 80 or 90% (on a molar basis) of all macromolecular species present. Most preferably, the object species is purified to essential homogeneity (contaminant species cannot be detected in the composition by conventional detection methods).

The term "ligand" as used herein refers to a molecule that is recognized by a particular receptor. The agent bound by or reacting with a receptor is called a "ligand," a term which is definitionally meaningful only in terms of its counterpart receptor. The term "ligand" does not imply any particular molecular size or other structural or compositional feature other than that the substance in question is capable of binding or otherwise interacting with the receptor. Also, a ligand may serve either as the natural ligand to which the receptor binds, or as a functional analogue that may act as an agonist or antagonist. Examples of ligands that can be investigated by this invention include, but are not restricted to, agonists and antagonists for cell membrane receptors, toxins and venoms, viral epitopes, hormones (for example, opiates, steroids, etc.), hormone receptors, peptides, enzymes, enzyme substrates, substrate analogs, transition state analogs, cofactors, drugs, proteins, and antibodies.

The term "linkage analysis" as used herein refers to a method of genetic analysis in which data are collected from affected families, and regions of the genome are identified that co-segregated with the disease in many independent families or over many generations of an extended pedigree. A disease locus may be identified because it lies in a region of the genome that is shared by all affected members of a pedigree. Methods of performing linkage analysis are disclosed, for example, in Sellick et al, Diabetes 52:2636-38 (2003), Sellick et al., Nucleic Acids Res., 32:e164 (2004), and Janecke et al., Nat. Genet., 36:850-4 (2004).

The term "linkage disequilibrium" or sometimes referred to as "allelic association" as used herein refers to the preferential association of a particular allele or genetic marker with a specific allele, or genetic marker at a nearby chromosomal location more frequently than expected by chance for any particular allele frequency in the population. For example, if locus X has alleles A and B, which occur equally frequently, and linked locus Y has alleles C and D, which occur equally frequently, one would expect the combination AC to occur with a frequency of 0.25. If AC occurs more frequently, then alleles A and C are in linkage disequilibrium. Linkage disequilibrium may result from natural selection of certain combination of alleles or because an allele has been introduced into a population too recently to have reached equilibrium with linked alleles. The genetic interval around a disease locus may be narrowed by detecting disequilibrium between nearby markers and the disease locus. For additional information on linkage disequilibrium see Ardlie et al., Nat. Rev. Gen. 3:299-309, 2002. Methods of performing genome wide association studies are disclosed, for example, in Hu et al., Cancer Res. 65:2542-6 (2005), Mitra et al., Cancer Res. 64:8116-25 (2004), Klein et al., Science 308:385-9 (2005) and Godde et al., J Mol. Med. 83:486-94 (2005).

The term "lod score" or "LOD" is the log of the odds ratio of the probability of the data occurring under the specific hypothesis relative to the null hypothesis. LOD=log [probability assuming linkage/probability assuming no linkage].

The term "mixed population" or sometimes refer by "complex population" as used herein refers to any sample containing both desired and undesired nucleic acids. As a non-limiting example, a complex population of nucleic acids may be total genomic DNA, total genomic RNA or a combination thereof. Moreover, a complex population of nucleic acids may have been enriched for a given population but include other undesirable populations. For example, a complex population of nucleic acids may be a sample which has been enriched for desired messenger RNA (mRNA) sequences but still includes some undesired ribosomal RNA sequences (rRNA).

The term "monomer" as used herein refers to any member of the set of molecules that can be joined together to form an oligomer or polymer. The set of monomers useful in the present invention includes, but is not restricted to, for the example of (poly)peptide synthesis, the set of L-amino acids, D-amino acids, or synthetic amino acids. As used herein, "monomer" refers to any member of a basis set for synthesis of an oligomer. For example, dimers of L-amino acids form a basis set of 400 "monomers" for synthesis of polypeptides. Different basis sets of monomers may be used at successive steps in the synthesis of a polymer. The term "monomer" also refers to a chemical subunit that can be combined with a different chemical subunit to form a compound larger than either subunit alone.

The term "mRNA" or sometimes refer by "mRNA transcripts" as used herein, include, but not limited to pre-mRNA transcript(s), transcript processing intermediates, mature mRNA(s) ready for translation and transcripts of the gene or genes, or nucleic acids derived from the mRNA transcript(s). Transcript processing may include splicing, editing and degradation. As used herein, a nucleic acid derived from an mRNA transcript refers to a nucleic acid for whose synthesis the mRNA transcript or a subsequence thereof has ultimately served as a template. Thus, a cDNA reverse transcribed from an mRNA, an RNA transcribed from that cDNA, a DNA amplified from the cDNA, an RNA transcribed from the amplified DNA, *etc*., are all derived from the mRNA transcript and detection of such derived products is indicative of the presence and/or abundance of the original transcript in a sample. Thus, mRNA derived samples include, but are not limited to, mRNA transcripts of the gene or genes, cDNA reverse transcribed from the mRNA, cRNA transcribed from the cDNA, DNA amplified from the genes, RNA transcribed from amplified DNA, and the like.

The term "nucleic acid library" or sometimes refer by "array" as used herein refers to an intentionally created collection of nucleic acids which can be prepared either synthetically or biosynthetically and screened for biological activity in a variety of different formats (for example, libraries of soluble molecules; and libraries of oligos tethered to resin beads, silica chips, or other solid supports). Additionally, the term "array" is meant to include those libraries of nucleic acids which can be prepared by spotting nucleic acids of essentially any length (for example, from 1 to about 1000 nucleotide monomers in length) onto a substrate. The term "nucleic acid" as used herein refers to a polymeric form of nucleotides of any length, either ribonucleotides, deoxyribonucleotides or peptide nucleic acids (PNAs), that comprise purine and pyrimidine bases, or other natural, chemically or biochemically modified, non-natural, or derivatized nucleotide bases. The backbone of the polynucleotide can comprise sugars and phosphate groups, as may typically be found in RNA or DNA, or modified or substituted sugar or phosphate groups. A polynucleotide may comprise modified nucleotides, such as methylated nucleotides and nucleotide analogs. The sequence of nucleotides may be interrupted by non-nucleotide components. Thus the terms nucleoside, nucleotide, deoxynucleoside and deoxynucleotide generally include analogs such as those described herein. These analogs are those molecules having some structural features in common with a naturally occurring nucleoside or nucleotide such that when incorporated into a nucleic acid or oligonucleoside sequence, they allow hybridization with a naturally occurring nucleic acid sequence in solution. Typically, these analogs are derived from naturally occurring nucleosides and nucleotides by replacing and/or modifying the base, the ribose or the phosphodiester moiety. The changes can be tailor made to stabilize or destabilize hybrid formation or enhance the specificity of hybridization with a complementary nucleic acid sequence as desired.

The term "nucleic acids" as used herein may include any polymer or oligomer of pyrimidine and purine bases, preferably cytosine, thymine, and uracil, and adenine and guanine, respectively. *See* Albert L. Lehninger, PRINCIPLES OF BIOCHEMISTRY, at 793-800 (Worth Pub. 1982). Contemplated is any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

The term "oligonucleotide" or sometimes refer by "polynucleotide" as used herein refers to a nucleic acid ranging from at least 2, preferable at least 8, and more preferably at least 20 nucleotides in length or a compound that specifically hybridizes to a polynucleotide. Polynucleotides include sequences of deoxyribonucleic acid (DNA) or ribonucleic acid (RNA) which may be isolated from natural sources, recombinantly produced or artificially synthesized and mimetics thereof. A further example of a polynucleotide may be peptide nucleic acid (PNA). Situations may occur in which there is a nontraditional base pairing such as Hoogsteen base pairing which has been identified in certain tRNA molecules and postulated to exist in a triple helix. "Polynucleotide" and "oligonucleotide" are used interchangeably in this application.

The term "polymorphism" as used herein refers to the occurrence of two or more genetically determined alternative sequences or alleles in a population. A polymorphic marker or site is the locus at which divergence occurs. Preferred markers have at least two alleles, each occurring at frequency of greater than 1%, and more preferably greater than 10% or 20% of a selected population. A polymorphism may comprise one or more base changes, an insertion, a repeat, or a deletion. A polymorphic locus may be as small as one base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allelic form is arbitrarily designated as the reference form and other allelic forms are designated as alternative or variant alleles. The allelic form occurring most frequently in a selected population is sometimes referred to as the wildtype form. Diploid organisms may be homozygous or heterozygous for allelic forms. A diallelic polymorphism has two forms. A triallelic polymorphism has three forms. Single nucleotide polymorphisms (SNPs) are included in polymorphisms.

The term "primer" as used herein refers to a single-stranded oligonucleotide capable of acting as a point of initiation for template-directed DNA synthesis under suitable conditions for example, buffer and temperature, in the presence of four different nucleoside triphosphates and an agent for polymerization, such as, for example, DNA or RNA polymerase or reverse transcriptase. The length of the primer, in any given case, depends on, for example, the intended use of the primer, and generally ranges from 15 to 30 nucleotides. Short primer molecules generally require cooler temperatures to form sufficiently stable hybrid complexes with the template. A primer need not reflect the exact sequence of the template but must be sufficiently complementary to hybridize with such template. The primer site is the area of the template to which a primer hybridizes. The primer pair is a set of primers including a 5' upstream primer that hybridizes with the 5' end of the sequence to be amplified and a 3' downstream primer that hybridizes with the complement of the 3' end of the sequence to be amplified.

The term "probe" as used herein refers to a surface-immobilized molecule that can be recognized by a particular target. See U.S. Patent Number 6,582,908 for an example of arrays having all possible combinations of probes with 10, 12, and more bases. Examples of probes that can be investigated by this invention include, but are not restricted to, agonists and antagonists for cell membrane receptors, toxins and venoms, viral epitopes, hormones (for example, opioid peptides, steroids, etc.), hormone receptors, peptides, enzymes, enzyme substrates, cofactors, drugs, lectins, sugars, oligonucleotides, nucleic acids, oligosaccharides, proteins, and monoclonal antibodies.

The term "receptor" as used herein refers to a molecule that has an affinity for a given ligand. Receptors may be naturally-occurring or manmade molecules. Also, they can be employed in their unaltered state or as aggregates with other species. Receptors may be attached, covalently or noncovalently, to a binding member, either directly or via a specific binding substance. Examples of receptors which can be employed by this invention include, but are not restricted to, antibodies, cell membrane receptors, monoclonal antibodies and antisera reactive with specific antigenic determinants (such as on viruses, cells or other materials), drugs, polynucleotides, nucleic acids, peptides, cofactors, lectins, sugars, polysaccharides, cells, cellular membranes, and organelles. Receptors are sometimes referred to in the art as anti-ligands. As the term receptors is used herein, no difference in meaning is intended. A "Ligand Receptor Pair" is formed when two macromolecules have combined through molecular recognition to form a complex. Other examples of receptors which can be investigated include but are not restricted to those molecules shown in U.S. Patent Number 5,143,854.

The term "solid support", "support", and "substrate" as used herein are used interchangeably and refer to a material or group of materials having a rigid or semi-rigid surface or surfaces. In many embodiments of the methods of the invention, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations. See U.S. Patent Number 5,744,305 for exemplary substrates.

The term "target" as used herein refers to a molecule that has an affinity for a given probe. Targets may be naturally-occurring or man-made molecules. Also, they can be employed in their unaltered state or as aggregates with other species. Targets may be attached, covalently or noncovalently, to a binding member, either directly or via a specific binding substance. Examples of targets include, but are not restricted to, antibodies, cell membrane receptors, monoclonal antibodies and antisera reactive with specific antigenic determinants (such as on viruses, cells or other materials), drugs, oligonucleotides, nucleic acids, peptides, cofactors, lectins, sugars, polysaccharides, cells, cellular membranes, and organelles. Targets are sometimes referred to in the art as anti-probes. As the term targets is used herein, no difference in meaning is intended. A "Probe Target Pair" is formed when two macromolecules have combined through molecular recognition to form a complex.

### c) Complexity Reduction by Target Specific Primer Extension and Affinity Purification

Generally, methods for amplifying genomic DNA for analysis are disclosed. Target specific primers are used to prime synthesis and a highly processive polymerase is used to extend the primers. The primers are extended to form long cDNAs that are preferably greater than 10,000 bases long and more preferably greater than 100,000 bases long. The amplified DNA may be fragmented, labeled and analyzed. The amplified DNA is analyzed by hybridization to an array of oligonucleotide probes. Disclosed is that the amplified DNA is analyzed by a locus specific genotyping method and a minimum number of locus specific primers are used to generate target suitable for downstream analysis, for example, mutation or polymorphism detection, genotyping, copy number analysis, translocation mapping or methylation analysis.

In general the methods allow targeted amplification of large regions of a genome, resulting in reduction of complexity and enrichment of target. The enriched target can then be analyzed for features of interest

All polymorphisms in a selected 10 Mb region of chromosome 1 that has been identified as part of a linkage peak may be genotyped. Using conditions that allow extension of a primer for up to 1,000,000 bases, 10 different primers could be used to amplify the region. If amplification conditions that allow extension of a primer for 100,000 bases then 100 different primers would be needed to amplify the 10 Mb region of interest. The amplified sample could be further amplified using, for example, MDA, methods disclosed in U.S. Patent Pub. No. 2003143599 and 2003040620, or any other non-specific amplification method. The amplified sample may then be analyzed by any method known to the art, for example, hybridization to a resequencing array, a genotyping array containing probes to interrogate known polymorphisms in the 10Mb region, all or a selected subset, or locus specific methods of genotyping methods, such as allele-specific PCR, allele specific SBE, allele specific ligation such as OLA, allele specific enzymatic cleavage, pyrosequencing, mass spectrometry and allele specific hybridization.

The target may be genotyped by allele specific hybridization of the target DNA to a high density SNP genotyping microarray. The amplified genomic DNA is also suitable for other methods of locus specific genotyping analysis. The amplified sample may be analyzed by any method known in the art, for example, MALDI-TOF mass spec, capillary electrophoresis, oligo ligation assay (OLA), dynamic allele specific hybridization (DASH) or TaqMan® (Applied Biosystems, Foster City, CA). For addition methods of genotyping analyses and references describing other methods *see* Syvanen, Nature Rev. Gen. 2:930-942 (2001) . The amplified DNA may also be used in genotyping methods such as those disclosed in Barker et al. Genome Res. 14:901-907 (2004).

In a preferred embodiment, the steps of the method include amplifying a region of DNA with labeled nucleotides, isolating the labeled target sequences or enriching for the labeled targets, hybridizing target sequences to a solid support, and analyzing hybridization patterns. One of the utilities of this method would be in selectively reducing complexity of the genome while dictating which portion of the genome to interrogate. Primers are designed to hybridize to specific targets so the products that will be amplified are predictable and determinable. A single primer is used to extend over a long range of sequence.

In one embodiment one or more locus-specific primers are annealed to genomic DNA and elongated using a DNA polymerase with a high processivity rate such as phi 29 DNA polymerase and Bst DNA polymerase (large fragment). The polymerase may extend the primer at least 100,000 bases and preferred the polymerase extends more than 1,000,000 bases. Ultra long extension may result in the use of a relatively small number of locus specific primers to generate amplification of one or more genomic regions of interest.

In another embodiment, *rTth* DNA polymerase, XL (eXtra Long) is used for primer extension over long ranges (available from Applied Biosystems). This themostable polymerase is designed for generating extra long PCR products. The enzyme is a specially formulated blend, capable of increased fidelity and high yield of long PCR products. The enzyme has both 5'-3' DNA polymerase activity and 3'-5' exonuclease (proofreading) activity. The r*Tth* DNA Polymerase, XL in XL PCR Buffer II was shown to amplify a 19.6 kb region of the beta-globin gene cluster from human genomic DNA and a 42 kb region from phage lambda DNA. See, Cheng, S., et al. 1994. Proc. Natl. Acad. Sci. USA 91:5,695-5,699 and Barnes, W.M. 1994. Proc. Natl. Acad. Sci. USA 91:2,216-2,220 for additional information about *rTth* polymerase. LA Taq (TaKaRa) is another thermal stable polymerase optimized for long extensions (greater than 15 kb).

The human genome is approximately 3 billion basepairs. Using extension of individual primers to about 100,000 bases it would require about 30 target specific primers to analyze 0.1% of the human genome or 3 million basepairs and 1% of the genome could be amplified using 300 target specific primers. It is estimated that SNPs occur on average about every 1,000 bases in the human genome so 300 primers extending for about 100,000 bases each would amplify 30,000,000 bases and should include about 30,000 SNPs.

The methods could also be used to analyze a smaller number of pre-selected regions. The regions could be, for example, resequenced in a plurality of individuals to identify novel polymorphisms or to determine the allele frequency of one or more polymorphisms in a population.

Often linkage or association studies result in the identification of large genomic regions that show linkage or association with a disease phenotype. Often these regions contain multiple target genes and may contain many known and unknown polymorphisms. To determine which gene and which polymorphism or polymorphisms are associated with the disease phenotype and which may be causing or contributing to the phenotype, the region must be analyzed at a more refined level. This may be accomplished by looking at more polymorphisms in the region and preferably by looking at all polymorphisms found in the identified region in the sample population. The methods presently disclosed may be used to amplify the region or regions identified by linkage or association so that those regions may be further analyzed to identify polymorphisms that are associated with the disease phenotype and to identify polymorphisms that may cause the disease phenotype.

Disclosed is that large scale mapping of disease loci may be performed using a fixed panel of SNPs that interrogate the entire genome at a selected resolution. Arrays capable of interrogating fixed SNP panels are available from Affymetrix and include, for example, the Mapping 10K array, the Mapping 100K array set (includes 2 50K arrays) and the Mapping 500K array set (includes two ~250K arrays). These arrays and array sets interrogate more than 10,000, 100,000 and 500,000 different human SNPs, respectively. The perfect match probes on the array are perfectly complementary to one or the other allele of a biallelic SNP. Each SNP is interrogated by a probe set comprising 24 to 40 probes. The perfect match probes in a probe set are each different, varying in, for example, the SNP allele, the position of the SNP relative to the center of the probe and the strand targeted. The probes are present in perfect match-mismatch pairs. The SNPs interrogated by a mapping array or array set are spaced throughout the genome with approximately equal spacing, for example, the SNPs in the 10K array are separated by about 200,000 base pairs. The median physical distance between SNPs in the 500K array set is 2.5 kb and the average distance between SNPs is 5.8 kb. Methods for using mapping arrays see, for example, Kennedy et al., Nat. Biotech. 21:1233-1237 (2003), Matsuzaki et al., Genome Res. 14:414-425 (2004), Matsuzaki et al., Nat. Meth. 1:109-111 (2004)Selected panels of SNPs can also be interrogated using a panel of locus specific probes in combination with a universal array as described in Hardenbol et al., Genome Res. 15:269-275 (2005) and in U.S. Patent No. 6,858,412. Universal tag arrays and reagent kits for performing such locus specific genotyping using panels of custom molecular inversion probes (MIPs) are available from Affymetrix.

Computer implemented methods for determining genotype using data from mapping arrays are disclosed, for example, in Liu, et al., Bioinformatics 19:2397-2403 (2003) and Di et al., Bioinformatics 21:1958-63 (2005). Computer implemented methods for linkage analysis using mapping array data are disclosed, for example, in Ruschendorf and Nurnberg, Bioinformatics 21:2123-5 (2005) and Leykin et al., BMC Genet. 6:7, (2005).

Methods for analyzing chromosomal copy number using mapping arrays are disclosed, for example, in Bignell et al., Genome Res. 14:287-95 (2004), Lieberfarb, et al., Cancer Res. 63:4781-4785 (2003), Zhao et al., Cancer Res. 64:3060-71 (2004), Nannya et al., Cancer Res. 65:6071-6079 (2005) and Ishikawa et al., Biochem. and Biophys. Res. Comm., 333:1309-1314 (2005).

Mapping analysis using fixed content arrays, for example, 10K, 100K or 500K arrays, preferably identify one or a few regions that show linkage or association with the phenotype of interest. Those linked regions may then be more closely analyzed to identify and genotype polymorphisms within the identified region or regions, for example, by designing a panel of MIPs targeting polymorphisms or mutations in the identified region. The targeted regions may be amplified by hybridization of a target specific primer and extension of the primer by a highly processive strand displacing polymerase, such as phi29 and then analyzed, for example, by genotyping.

Target amplification by the disclosed methods may be used for array-based sequencing applications. The sequence of a nucleic acid may be compared to a known reference sequence by hybridization to an array of probes that detects all possible single nucleotide variations in the reference sequence. Such arrays, known as resequencing arrays, are commercially available from Affymetrix, Inc. and have been described, for example, see Cutler, D. J. et al. Genome Research 11(11), 1913-25, 2001. During sample preparation for resequencing analysis target sequences are amplified. This has typically done by PCR amplification using pairs of primers that are specific, for segments of the target to be analyzed. PCR amplification has typically been performed using long range PCR in order to maximize the length of the PCR amplicons. This still requires multiple different PCR reactions which are then pooled prior to analysis, often requiring quantification of the amplicons in order to facilitate pooling of approximately equal amounts. Resequencing arrays may be used to analyze both strands of 30 kb or more and 300 kb or more to detect polymorphisms in the sample sequence compared to a reference sequence. Instead of amplification by PCR, the target may be amplified by long range amplification using a strand displacing enzyme such as Phi 29 or Bst DNA polymerase, as disclosed herein. For example, a single primer may be used to prime synthesis at a specific locus and extend through the 30-300 kb of target sequence to be analyzed.

In another embodiment DNA that has been amplified by locus specific amplification may be subjected to a second round of amplification using a second method of amplification, for example, multiple strand displacement. The second round of amplification increases the overall mass of the selected fragments prior to fragmentation, labeling, and hybridization. For a description of multiple displacement assay, see for example Lasken and Egholm, Trends Biotechnol. 2003 21(12):531-5; Barker et al. Genome Res. 2004 May;14(5):901-7; Dean et al. Proc Natl Acad Sci U S A. 2002; 99(8):5261-6; and Paez, J.G., et al. Nucleic Acids Res. 2004; 32(9):e71.

In one embodiment biotinylated nucleotides may be incorporated during elongation so that freshly prepared single stranded DNA will have incorporated biotin. In another embodiment dNTPs labeled with digoxigenin labeled dNTPs may be used. In another aspect a primer comprising a 5' biotin may be used for extension. After extension, those primers that have not been extended may be separated from the extension products, for example, by size based separation.

In one embodiment a thermal stable polymerase is used and the resulting duplexes may be denatured and multiple rounds of annealing, elongation and denaturation may be performed. Linear extension of the desired genomic regions will result and the overall mass of the extension product will be increased. Since a second strand primer is not present, such as for PCR, exponential amplification should be largely absent.

Newly extended strands may be selected by incubation with streptavidin coated beads which may be magnetic or an anti-biotin antibody conjugated to a solid support, for example, agarose.

As defined in the claims, target amplification according to the disclosed methods is used to assess chromosomal translocations. In one embodiment primer is annealed upstream of the site of a known translocation and elongated through the translocation, affinity labels may be incorporated into the amplified target to facilitate enrichment of amplified target. The amplified target may be hybridized to arrays that have probes for both chromosomes known to be involved in the translocation. The hybridization pattern may be analyzed to identify probes where hybridization signal is present.

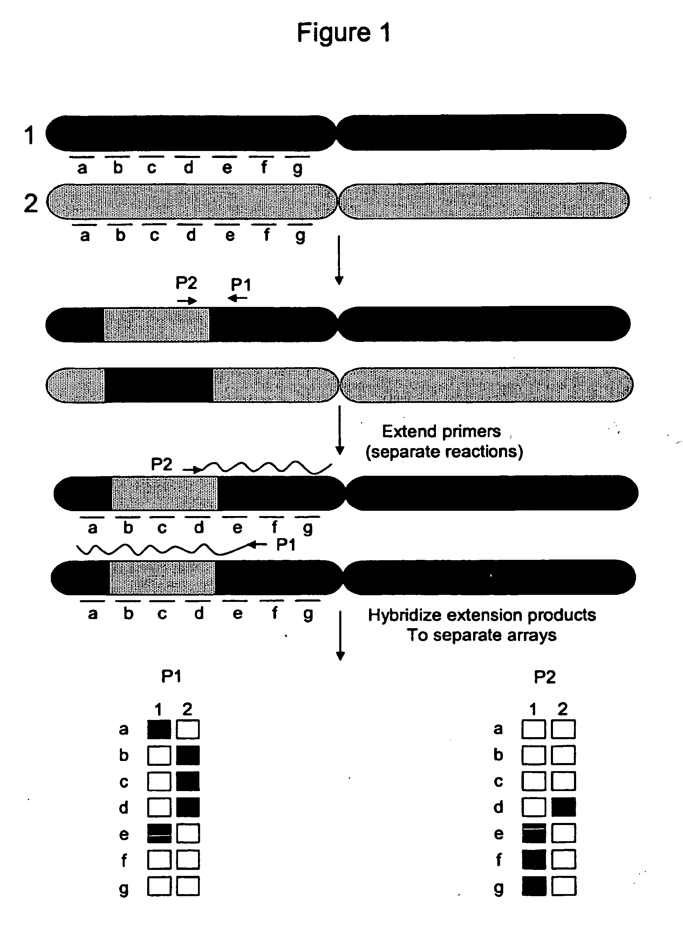

Figure 1 shows a schematic of one embodiment of the method. Chromosomes 1 and 2 are known to be involved in a reciprocal translocation in some cancers. A DNA sample suspected of containing the translocation is contacted separately with either a primer to chromosome 1 that hybridizes upstream of the translocation (P1) and P1 is extended. In a separate reaction a primer that hybridizes to chromosome 2 in the region that is known to be translocated (P2) is hybridized to the sample and extended. The reactions are separately hybridized to an array of probes comprising a plurality of probes to chromosome 1 and chromosome 2 (a1-g1 and a2-g2). In the reaction where P1 was extended the extension product from the translocation hybridizes to probes a1, b2, c2, d2, and e1. In the reaction where P2 was extended the extension product from the translocation hybridizes to probes d2, e1, fl and g1. The translocation breakpoint can be mapped to the region between probes d and e. The resolution of mapping will depend on the distance between probes d and e. In some embodiments probes are tiled so that every base is interrogated so the mapping can determine the exact position of the translocation breakpoint. Wider spacing of probes is also possible, for example every 10 to 100 bases. In some embodiments an array may comprise probes that are more densely spaced at known regions of translocations, for example, a region that is known to be a breakpoint for a known translocation may be targeted by probes that are tiled to interrogate every base while regions that are typically not close to a breakpoint are tiled to interrogate every 10 to 100 bases.

If the translocation is not present hybridization should be observed for the first chromosome but not the second. If the translocation is present hybridization should be observed to probes for both chromosomes involved in the translocation. The process may then be repeated using a primer that is complementary to the second chromosome. The translocation breakpoint may be identified by mapping the probes that show hybridization. For translocation analysis an array that has probes tiled along chromosomal regions may be used. The probes may be placed at common intervals along the region of interest, for example, every 2, 5, 25, 35, 50 or 100 bases or the probes may be tiled to interrogate every base. Probes that are complementary to the junction created by a translocation may also be included. Translocation junction probes would only show specific hybridization if the translocation is present.

Amplification consists of annealing at least one locus specific primer to double stranded DNA and elongating using a DNA polymerase with a high processivity rate, such as phi 29 DNA polymerase or Bst DNA polymerase. In a preferred embodiment at least 10, 25, 100 or 1000 locus specific primers are used. The region of DNA that is amplified preferably comprises at least one polymorphic locus. In a preferred embodiment the region that is amplified using each locus specific primer contains more than 5, 10, 15, 50 or 100 polymorphisms. In one embodiment, the polymerase extends at least 100,000, 200,000, 500,000 or 700,000 base pairs or more. In a preferred embodiment, the polymerase extends up to about 1,000,000 base pairs or more. Ultra long extension requires fewer primers to generate the portion or portions of the genome of interest.

In some embodiments labeled nucleotides are incorporated into the amplified DNA products by the DNA polymerase to form labeled target sequences. In a preferred embodiment of the methods, biotinylated nucleotides will be incorporated during elongation such that only freshly prepared single stranded DNA will be labeled to produce biotinylated target sequences. In another embodiment of the methods, nucleotides labeled with digoxigenin can be used. The newly synthesized DNA may be affinity purified. Methods of affinity purification of nucleic acids are described in U.S. Patent Nos. 6,013,440, 6,280,950, and 6,440,677.

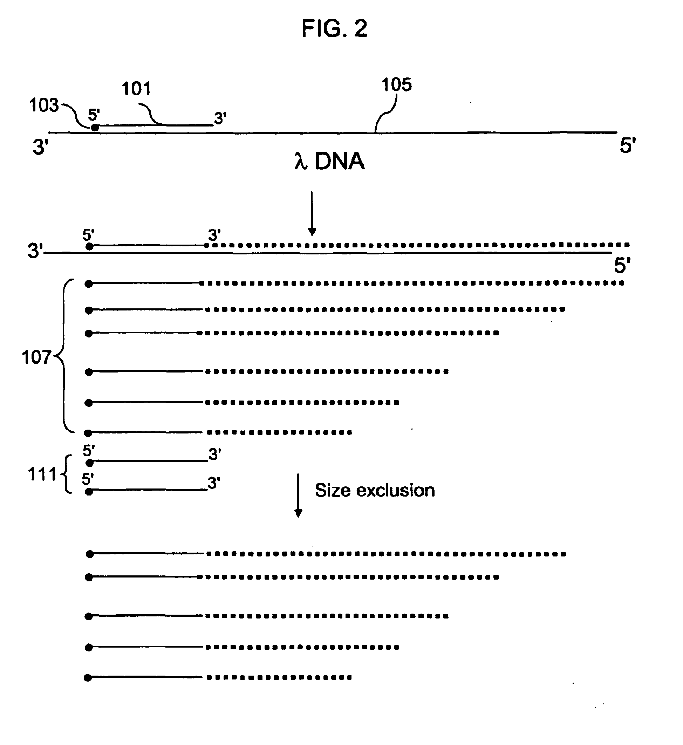

In another embodiment a primer (101) labeled with an affinity label (103) such as photocleavable biotin is used in the extension reaction (Fig. 2). The primer is complementary to the template (105) and is extended to generate extension products (107) that have the affinity label at the 5' end. The unextended primers (111) can be removed by size exclusion chromatography, for example, by passing the reaction over an S-400 column. The remaining extension products may then be affmity purified. In one embodiment the affinity label on the primer is biotin and the extension products are subsequently immobilized to a solid support such as beads, for example, DYNABEADS coated with Streptavidin. In preferred embodiments magnetic beads coated with streptavidin or avidin are used to separate primer extension products from unlabeled nucleic acids in the sample. The primer extension product immobilized to the bead can then be extensively washed to remove the template nucleic acid and then the extended DNA can be eluted from the solid support, for example, by photocleavage. Photocleavable biotin derivatives and a photocleavable phosphoramidite (PCB-phosphoramidite) are disclosed in Olejnik et al., Nuc. Acids Res. 24:361-6 (1996) and Olejnik et al., PNAS 92:7590-4 (1995). Also disclosed in these publications are methods of using the PCB moiety for purification of nucleic acids. The biotin moiety is linked by a spacer to a photocleavable moiety. PCB-phosphoramidite can be used to introduce a photocleavalbe biotin label (PCB) to the 5' terminal phosphate of a synthetic oligonucleotide. Biotin has a very strong affinity toward avidin/streptavidin, making elution difficult. In contrast, photocleavage allows efficient and rapid release of the nucleic acid. Release occurs efficiently by irradiation with 300-350 nm light.

The eluted extension products may then be subjected to a second round of amplification, for example, by MDA or by Rubicon's WGA methods. The product which is enriched for the desired fragments may then be analyzed by hybridization to an array.In a preferred embodiment, a thermal stable enzyme is used and resulting duplexes may be denatured, for example, by heat, and subjected to denaturing multiple rounds of annealing, elongating, and denaturing. This may be used to increase the overall mass of the extension products without resulting in an exponential amplification since there is no primer present that targets the opposite strand of DNA as in PCR.

Isolating the labeled target sequences consists of incubating for selection of the labeled target sequences, fragmenting the selected target sequences, end-labeling the selected target sequences, and performing multiple strand displacement assay. In some embodiments of the methods, the newly extended strands are selected by incubation with streptavidin coated magnetic beads. In some embodiments of the methods, the newly extended strands are selected by incubation with an anti-biotin antibody conjugated to agarose. If this approach is used in conjunction with Multiplexed Anchored Run-off Amplification (MARA), using a restriction enzyme that cuts infrequently, such as Not I, other means of purification of newly synthesized DNA to be used, such as digestion with T7 Gene 6 exonuclease (or another exonuclease that cleaves 5' to 3' but not 3' to 5') in conjunction with locus specific primers modified with phophorothiate linkages at the 5' end. MARA methods are disclosed in US Patent Application Nos. 2003/232348 and 2005/142577.

The selected target sequences may be subjected to multiple strand displacement assay using a phi29 polymerase and exonuclease-protected random hexamer primers. The amplified sample is subjected to exonuclease digestion with an exonuclease that digests in a 5' to 3' direction but not 3' to 5'. Newly synthesized DNA is protected from digestions so the sample is enriched for newly synthesized DNA after digestion. The sample may be analyzed as described above. The extended fragments are hybridized to an array of probes and the labeled nucleotides or nucleotides present at each location are determined.

In one embodiment of the methods of the invention, the solid support is a high density array. In another embodiment of the methods, the solid support is a microtiter dish. In another embodiment of the methods, the solid support is beads. The target sequences are hybridized to at least two probes that are immobilized to known locations on the solid support. The first probe is complementary to the first allelic form of at least one of the polymorphic locus. The second probe is complementary to the second allelic form of at least one of the polymorphic locus. Methods of probe array use are described in U.S. Patent Nos. 5,837,832, 6,156,501, and 6,368,799

Analyzing the pattern of hybridization consists of detecting the presence or absence of an allele. A labeled antibody is used to detect labeled probe-target complexes. In some embodiments, the antibody is an anti-streptavidin antibody, used to detect biotin on the probe-target complex on the solid support. If there is hybridization of the target sequence to the probe then the probe-target complex will be biotinylated. Methods of use for polymorphisms and SNP discovery can be found, for example in U.S. Pat. No. 6,361,947.

Polymerases useful in this method include those that are highly processive and strand displacing, such as Phi29 and Bst DNA polymerase (large fragment). The polymerase preferably should displace the polymerized strand downstream from the nick, and preferably lacks substantial 5' to 3' exonuclease activity. Enzymes that may be used include, for example, the Klenow fragment of DNA polymerase I, Bst polymerase large fragment, Phi29 and others. DNA Polymerase I Large (Klenow) Fragment consists of a single polypeptide chain (68kDa) that lacks the 5'->3' exonuclease activity of intact *E. coli* DNA polymerase I, but retains its 5'->3' polymerase, 3'->5' exonuclease and strand displacement activities. The Klenow fragment has been used for strand displacement amplification (SDA). See, e.g., U.S. Patent Nos. 6,379,888; 6,054,279; 5,919,630; 5,856,145; 5,846,726; 5,800,989; 5,766,852; 5,744,311;5,736,365; 5,712,124; 5,702,926; 5,648,211;5,641,633; 5,624,825; 5,593,867; 5,561,044; 5,550,025; 5,547,861; 5,536,649; 5,470,723; 5,455,166; 5,422,252; 5,270,184. SDA is an isothermal in vitro method for amplification of nucleic acid. SDA initiates synthesis of a copy of a nucleic acid at a free 3' OH that may be provided, for example, by a primer that is hybridized to the template. The DNA polymerase extends from the free 3' OH and in so doing displaces the strand that is hybridized to the template leaving a newly synthesized strand in its place. Repeated nicking and extension with continuous displacement of new DNA strands results in exponential amplification of the original template.

Phi29 is another DNA polymerase with high strand displacing activity. Phi29 is a highly processive enzyme that is capable of extending long regions of DNA, for example, 100 kb or longer fragments. Variants of phi29 enzymes may be used, for example, an exonuclease minus variant may be used. For additional information on phi29 see, for example, U.S. Patent Nos. 5,100,050, 5,198,543 and 5,576,204.

Bst DNA polymerase is another polymerase that is known to have strand displacing activity. The enzyme is available from, for example, New England Biolabs. Bst is active at high temperatures and the reaction may be incubated, for example at about 65°C. The enzyme tolerates reaction conditions of 70°C and below and can be heat inactivated by incubation at 80°C for 10 minutes. For additional information see Mead, D.A. et al. (1991) BioTechniques , p.p. 76-87, McClary, J. et al. (1991) J. DNA Sequencing and Mapping , p.p. 173-180 and Hugh, G. and Griffin, M. (1994) PCR Technology , p.p. 228-229.

Examples of other polymerases with strand displacing activity include: exo minus Vent (NEB), exo minus Deep Vent (NEB), Bst (BioRad), exo minus Pfu (Stratagene), Pfx (Invitrogen), 9°Nₘ™ (NEB), Bca (Panvera), and other thermostable polymerases. Other characteristics of strand displacing enzymes that may be taken into consideration are described, for example, in US Patent No. 6,692,918.

As defined in the claims, the disclosed methods are used to detect chromosomal translocations. A chromosomal translocation results when two previously unlinked segments of the genome are brought together. In some cases translocation can result in disease by inducing inappropriate expression of a protein or synthesis of a new fusion protein. This phenomenon is particularly important when the breakpoint of the translocation affects an oncogene and results in cancer.

Specific translocations have been identified and associated with particular phenotypes. For example, chronic myeloid leukemia (CML) is caused by a specific translocation. This translocation was shown to involve reciprocal fusion of small pieces from the long arms of chromosome 9 and 22. The altered, abnormally short chromosome 22 that results is known as the Philadelphia chromosome (abbreviated as Ph). In the formation of the Ph translocation, two fusion genes are generated: BCR-ABL on the Ph chromosome and ABL-BCR on the chromosome 9 participating in the translocation. The bcr-abl fusion gene encodes a phosphoprotein (p210) that functions as a disregulated protein tyrosine kinase and predisposes the cell to become neoplastic.

Another well studied example of a translocation generating cancer is seen in Burkitt's lymphoma. In most cases of this B cell tumor, a translocation is seen involving chromosome 8 and one of three other chromosomes (2, 14 or 22). In these cases, a fusion protein is not produced, but rather, the c-myc proto-oncogene on chromosome 8 is brought under transcriptional control of an immunoglobulin gene promoter. In B cells, immunoglobulin promoters are transcriptionally quite active, resulting in overexpression of c-myc, which is known from several other systems to have oncogenic properties. Hence, this translocation results in aberrant high expression of an oncogenic protein, which almost certainly is at the root of the Burkitt's tumor. There are about 70 translocations that have been identified. Other examples of translocation breakpoints associated with human cancer include: 14:18 translocation in follicular B cell lymphomas (bcl-2 and immunoglobulin genes); 15:17 translocation in acute promyelocytic leukemia (pml and retinoic acid receptor genes) and 1:19 translocation in acute pre-B cell leukemia (PBX-1 and E2A genes).

Chromosomal abnormalities can be classified into two types according to the extent of their occurrence in the body. A constitutional abnormality is present in all cells of the body and a somatic or acquired abnormality is present in only certain cells or tissues, a condition known as mosaicism. Structural chromosomal abnormalities can result from misrepair of chromosome breaks or recombination between non homologous chromosomes. Aneuploidy is when one or more individual chromosomes is present in an extra copy or is missing from a euploid set. Trisomy means having three copies of a particular chromosome in an otherwise diploid cell. Cancer cells often show extreme aneuploidy. Two main mechanisms are responsible for most aneuploidy: nondisjucntion and anaphase lag. Other chromosomal abnormalities that may be detected by the methods include paracentric inversions, interstitial deletions and ring chromosome formation.

Chromosomal breaks can cause a loss-of-function phenotype if it disrupts the coding sequence of a gene, or separates it from a nearby regulatory regions. It can also cause a gain of function, for example by splicing exons of two genes together to create a novel chimeric gene, which is common in tumorigensesi. Breakpoints provide valuable clues to the exact physical location of a disease gene. The precise position of the breakpoint may be defined by the presently disclosed methods.

Different types of known translocations that may be detected, for example, include reciprocal translocations, Robertsonian translocations, deletions, pericentric inversions, paracentric inversions, insertions, and ring chromosome formation.

An insertion translocation results when an interstitial segment of a first chromosome is deleted and transferred to a new position in a second chromosome, or occasionally, into its homologue or somewhere else within the same chromosome. The inserted segment may be positioned with its original orientation with respect to the centromere or it may be inverted. This is usually a balanced rearrangement without loss of genetic information.

Insertions may be detected by the presently disclosed methods. When a primer that is complementary to a region that is within the segment of the first chromosome that is transferred to the second chromosome is extended, the primer may be extended along the translocated region of the first chromosome, through one of the breakpoints and into the second chromosome. The primer extension product will have sequence from both the first and second chromosomes and when the primer extension product is fragmented and labeled fragments will hybridize to probes that are complementary to the first chromosome and probes that are complementary to the second chromosome. The breakpoint may also be detected. Probes that are upstream of the breakpoint should not show hybridization while probes that are downstream of the breakpoint will show hybridization.

### d) Examples

### Example 1. Biotinylated Nucleotide Incorporation

Reaction mixtures were set up with the following: 53 µl water, 30 µl 3.3X XL Buffer II, 2 µl 50X dNTP mix, 1.6 µl primer SC1011, 1.6 µl primer SC1002, 4.8 µl Mg(OAc)₂, 1.0 µl Lambda DNA, 4 µl 1 mM Biotin-dNTP and 2.0 µl rTth polymerase. The fmal concentrations in the reaction are 1.2 mM MgOAc, 4 Units rTth and 40 pmol each of the primers. The primers amplify a 20.8 kb product from lambda DNA. Individual 50X dNTP mixes were made for each biotin-dNTP that was tested. The 50X ACGT mix contained 8 µl 100mM dATP, 10 µl each of 100mM dCTP, dGTP, TTP, and water up to a volume of 100 µl. This mix is then used in conjunction with biotin-dATP so that the PCR reaction contains a mixture of cold dATP and biotin-dATP.

The reactions were incubated for 1 min at 94°C, then 16 cycles of: 94°C for 15 sec and 10 min at 68°C; 12 cycles of: 94°C for 15 sec and 10 min at 68°C (increment = 15 sec per cycle); and 1 cycle of 72° for 10 min and hold at 4°C.

The depletion experiments were done using a monoclonal anti-biotin-agarose Clone BN-34 from Sigma (Product No. A1559). The PCR reactions were passed over G-25 Sephadex columns to remove unincorporated biotin-dNTPs. The anti-biotin-agarose is then added to the PCR product and incubated at room temp for 15-30 min with gentle agitation in a buffered solution (such as TE or 1X PCR buffer).

Reactions 1-4 contain 40 µM (final) of the biotinylated nucleotide, for example dATP plus 160 µM (final) of the unlabeled nucleotides, for example dATP. The other three unlabeled nucleotides were present in a final concentration of 200 µM. Reactions were cycled and an aliquot was run on 2% agarose 1X TBE gel. A positive control of standard dNTP and a negative control of no dNTP added to PCR mixture were also run on the 2% agarose 1X TBE gel. The results show that biotin dATP, biotin dCTP, biotin dGTP, and biotin dUTP were incorporated.

### Example 2. Depletion of Control DNA Fragments with Monoclonal Anti-Biotin Agarose

PCR fragments were amplified from human genomic DNA using various primer pairs. An aliquot of each reaction was run on a 2% agarose 1X TBE gel. Individual tubes containing the various PCR products were set up and an aliquot was taken of each sample prior to the addition of monoclonal anti-biotin agarose. Monoclonal anti-biotin agarose was added and the samples were incubated at R for 15 minutes with periodic gentle agitation. The samples were centrifuged at 5000 rpm for 3 minutes to pellet the agarose. The supernatant was recovered and an aliquot was run on a 2% agarose 1X TBE gel. The results show that there is preferential depletion of biotinylated PCR products by anti-biotin-agarose. The biotinylated fragments were all as bright as or brighter than the standard primers in the pre-depletion gel picture. The biotinylated fragments were all dimmer than the standard primers in the post-depletion gel.

### Example 3. PCB-labeled primer extension and photocleavage.

A primer labeled at the 5' end with a photocleavable biotin moiety was used in a primer extension reaction using lambda DNA as template. The single primer was used in a series of cycles of heating, annealing, and extension. Unextended primers were removed by passing the reaction over an S-400 column. Biotinylated fragments were immobilized by binding to streptavidin DNYABEADS. The bound fragments were washed under stringent conditions and released from the beads by photocleavage. The released fragment was tested by PCR to determine which regions of the starting template (lambda DNA) were copied. Eight primer pairs were tested and all but one gave the expected product, indicating that the extension products of about 45 kb were generated. Release was by UV irradiation at 0 or 15 cm distance and 1 or 5 minutes of exposures.

### Example 4.

LA Taq and Bst DNA Pol were tested with either 10 target specific primers or no primer. For LA Taq a 2 step thermal cycling procedure was used. For Bst DNA Pol isothermal amplification at 65°C was used. Products were captured using streptavidin coated magnetic beads with stringent washing, including washes with 0.15 N NaOH.

## Claims

1. A method of detecting a translocation between a first and a second chromosome comprising:
contacting a nucleic acid sample with a first primer that is complementary to the first chromosome and extending said first primer to form first primer extension products;
labeling said first primer extension products;
hybridizing said labeled first primer extension products to an array comprising a plurality of probes for said first chromosome and a plurality of probes for said second chromosome to obtain a hybridization pattern;
analyzing said hybridization pattern wherein the presence of hybridization to probes for said second chromosome is indicative of the presence of a translocation between said first and second chromosomes.

2. The method of claim 1 further comprising contacting the sample with a second primer that is complementary to the second chromosome and extending the second primer to form second primer extension products;
labeling said second primer extension products;
hybridizing said labeled second primer extension products to an array comprising a plurality of probes for said first chromosome and a plurality of probes for said second chromosome to obtain a hybridization pattern;
analyzing said hybridization pattern wherein the presence of hybridization to one or more probes for said first chromosome is indicative of the presence of a translocation between said first and second chromosomes.

3. The method of claim 1 or 2 wherein the translocation being detected is a known translocation and wherein the first primer is selected to be complementary to an area that is unchanged in the first chromosome but is near one of the breakpoints of said known translocation.

4. The method of claim 2 wherein the translocation being detected is a known translocation and wherein the first primer is complementary to an area that is unchanged in the first chromosome but is near a breakpoint of the translocation and wherein the second primer is complementary to a region of the second chromosome that is translocated into the first chromosome.

5. The method of claim 1 wherein said extending step comprises nucleotides comprising an affinity label and wherein said nucleotides are incorporated into the extension products to obtain affinity labeled extension products.

6. The method of claim 1 wherein said extending step comprises a biotinylated dNTP that is incorporated into the extension products.

7. The method of claim 6 wherein the biotinylated dNTP is biotin-dUTP.

8. The method of claim 1 wherein said extending step uses a DNA polymerase selected from the group consisting of phi29 DNA polymerase, Bst DNA polymerase, LA Taq and rTth DNA polymerase.

9. The method of claim 1 wherein the extending step comprises a digoxigenin labeled dNTP that is incorporated into the extension products.

10. The method of claim 1 wherein the first primer is extended at least about 10,000 bases.

11. The method of claim 1 wherein the first primer is extended about 100,000 bases.

12. The method of claim 1 wherein said first primer is extended through a region comprising between 50 and 1,000 polymorphisms.

13. The method of claim 1 wherein said plurality of probes are attached to a solid support.

14. The method of claim 1 wherein said plurality of probes are oligonucleotide probes that are between 20 and 80 bases in length and wherein said array comprises at least 500,000 different probes that are present at known or determinable locations.

15. The method of claim 6 further comprising affinity purification of the primer extension products, comprising incubation of the primer extension products with anti-biotin antibody conjugated to agarose to bind the primer extension products to the agarose and removal of unbound nucleic acid.

16. The method of claim 8 wherein said polymerase is phi29 DNA polymerase.

17. The method of claim 1 wherein said first primer is resistant to 5' to 3' exonuclease digestion and said method further comprising digesting the primer extension products generated with a 5' to 3' exonuclease.

18. The method of claim 8 wherein said DNA polymerase is Bst DNA polymerase.

## Patentansprüche

1. Ein Verfahren zum Detektieren einer Translokation zwischen einem ersten und einem zweiten Chromosom, umfassend:
das in Kontakt bringen einer Nukleinsäureprobe mit einem ersten Primer, der zu dem ersten Chromosom komplementär ist, und das Verlängern des ersten Primers, um erste Primerverlängerungsprodukte zu bilden;
das Markieren der ersten Primerverlängerungsprodukte;
das Hybridisieren der markierten ersten Primerverlängerungsprodukte mit einem Array, das eine Mehrzahl von Sonden für das erste Chromosom und
eine Mehrzahl von Sonden für das zweite Chromosom umfasst, um ein Hybridisierungsmuster zu eralten;
das Analysieren des Hybridisierungsmusters, wobei das Vorliegen einer Hybridisierung mit Sonden für das zweite Chromosom ein Hinweis auf das Vorliegen einer Translokation zwischen dem ersten und zweiten Chromosom ist.

2. Das Verfahren nach Anspruch 1, welches weiterhin umfasst: das in Kontakt bringen der Probe mit einem zweiten Primer, der zu dem zweiten Chromosom komplementär ist, und das Verlängern des zweiten Primers, um zweite Primerverlängerungsprodukte zu bilden;
das Markieren der zweiten Primerverlängerungsprodukte;
das Hybridisieren der markierten zweiten Primerverlängerungsprodukte mit einem Array, das eine Mehrzahl von Sonden für das erste Chromosom und eine Mehrzahl von Sonden für das zweite Chromosom umfasst, um ein Hybridisierungsmuster zu erhalten;
das Analysieren des Hybridisierungsmusters, wobei das Vorliegen einer Hybridisierung mit einer oder mehreren Sonden für das erste Chromosom ein Hinweis auf das Vorliegen einer Translokation zwischen dem ersten und zweiten Chromosom ist.

3. Das Verfahren nach Anspruch 1 oder 2, wobei die Translokation, die detektiert wird, eine bekannte Translokation ist und wobei der erste Primer so ausgewählt wird, dass dieser zu einem Bereich komplementär ist, der in dem ersten Chromosom unverändert ist, sich aber nahe an einem der Bruchpunkte der bekannten Translokation befindet.

4. Das Verfahren nach Anspruch 2, wobei die Translokation, die detektiert wird, eine bekannte Translokation ist und wobei der erste Primer zu einem Bereich komplementär ist, der in dem ersten Chromosom unverändert ist, sich aber nahe an einem Bruchpunkt der Translokation befindet, und wobei der zweite Primer zu einer Region des zweiten Chromosoms komplementär ist, die in das erste Chromosom transloziert ist.

5. Das Verfahren nach Anspruch 1, wobei der Verlängerungsschritt Nukleotide umfasst, die eine Affinitätsmarkierung umfassen, und wobei die Nukleotide in die Verlängerungsprodukte eingebaut werden, um affinitätsmarkierte Verlängerungsprodukte zu erhalten.

6. Das Verfahren nach Anspruch 1, wobei der Verlängerungsschritt ein biotinyliertes dNTP umfasst, das in die Verlängerungsprodukte eingebaut wird.

7. Das Verfahren nach Anspruch 6, wobei das biotinylierte dNTP Biotin-dUTP ist.

8. Das Verfahren nach Anspruch 1, wobei der Verlängerungsschritt eine DNA-Polymerase verwendet, die ausgewählt wird aus der Gruppe, bestehend aus phi29-DNA-Polymerase, Bst-DNA-Polymerase, LA-Taq- und rTth-DNA-Polymerase.

9. Das Verfahren nach Anspruch 1, wobei der Verlängerungsschritt ein mit Digoxigenin markiertes dNTP umfasst, das in die Verlängerungsprodukte eingebaut wird.

10. Das Verfahren nach Anspruch 1, wobei der erste Primer um wenigstens ca. 10.000 Basen verlängert wird.

11. Das Verfahren nach Anspruch 1, wobei der erste Primer um ca. 100.000 Basen verlängert wird.

12. Das Verfahren nach Anspruch 1, wobei der erste Primer über eine Region hinweg verlängert wird, die zwischen 50 und 1.000 Polymorphismen umfasst.

13. Das Verfahren nach Anspruch 1, wobei die Mehrzahl von Sonden an einem festen Träger befestigt ist.

14. Das Verfahren nach Anspruch 1, wobei die Mehrzahl von Sonden Oligonukleotidsonden sind, die eine Länge zwischen 20 und 80 Basen aufweisen, und wobei das Array wenigstens 500.000 verschiedene Sonden umfasst, die an bekannten oder bestimmbaren Orten vorliegen.

15. Das Verfahren nach Anspruch 6, welches weiterhin eine Affinitätsreinigung der Primerverlängerungsprodukte, die eine Inkubation der Primerverlängerungsprodukte mit einem anti-Biotin-Antikörper umfasst, der mit Agarose konjugiert ist, um die Primerverlängerungsprodukte an die Agarose zu binden, und die Entfernung von ungebundener Nukleinsäure umfasst.

16. Das Verfahren nach Anspruch 8, wobei die Polymerase phi29-DNA-Polymerase ist.

17. Das Verfahren nach Anspruch 1, wobei der erste Primer gegenüber einem Verdau mit 5'-nach-3'-Exonuklease beständig ist, und das Verfahren weiterhin das Verdauen der Primerverlängerungsprodukte, die mit einer 5'-nach-3'-Exonuklease erzeugt wurden, umfasst.

18. Das Verfahren nach Anspruch 8, wobei die DNA-Polymerase Bst-DNA-Polymerase ist.

## Revendications

1. Procédé de détection d'une translocation entre un premier et un second chromosome, comprenant :
la mise en contact d'un échantillon d'acide nucléique avec une première amorce qui est complémentaire au premier chromosome et l'extension de ladite première amorce afin de former des premiers produits d'extension d' amorce ;
le marquage desdits premiers produits d'extension d'amorce ;
l'hybridation desdits premiers produits d'extension d'amorce marqués à un réseau comprenant une pluralité de sondes pour ledit premier chromosome et une pluralité de sondes pour ledit second chromosome afin d'obtenir un schéma d'hybridation ;
l'analyse dudit schéma d'hybridation, où la présence d'une hybridation à des sondes pour ledit second chromosome indique la présence d'une translocation entre ledit premier et second chromosome.

2. Procédé selon la revendication 1, comprenant en outre la mise en contact de l'échantillon avec une seconde amorce qui est complémentaire au second chromosome et l'extension de la seconde amorce afin de former des seconds produits d'extension d'amorce ;
le marquage desdits seconds produits d'extension d'amorce ;
l'hybridation desdits seconds produits d'extension d'amorce marqués à un réseau comprenant une pluralité de sondes pour ledit premier chromosome et une pluralité de sondes pour ledit second chromosome afin d'obtenir un schéma d'hybridation ;
l'analyse dudit schéma d'hybridation, où la présence d'une hybridation à une ou plusieurs sondes pour ledit premier chromosome indique la présence d'une translocation entre ledit premier et second chromosome.

3. Procédé selon la revendication 1 ou 2, dans lequel la translocation qui est détectée est une translocation connue et dans lequel la première amorce est sélectionnée pour être complémentaire à une région qui n'est pas modifiée dans le premier chromosome mais qui est proche d'un des points de cassure de ladite translocation connue.

4. Procédé selon la revendication 2, dans lequel la translocation qui est détectée est une translocation connue et dans lequel la première amorce est complémentaire à une région qui n'est pas modifiée dans le premier chromosome mais qui est proche d'un point de cassure de la translocation, et dans lequel la seconde amorce est complémentaire à une région du second chromosome qui subit une translocation dans le premier chromosome.

5. Procédé selon la revendication 1, dans lequel ladite étape d'extension comprend des nucléotides comprenant un marqueur d'affinité et dans lequel lesdits nucléotides sont incorporés dans les produits d'extension afin d'obtenir des produits d'extension marqués par affinité.

6. Procédé selon la revendication 1, dans lequel ladite étape d'extension comprend un dNTP biotinylé qui est incorporé dans les produits d'extension.

7. Procédé selon la revendication 6, dans lequel le dNTP biotinylé est un dUTP-biotine.

8. Procédé selon la revendication 1, dans lequel ladite étape d'extension utilise une ADN polymérase sélectionnée dans le groupe comprenant l'ADN polymérase phi29, l'ADN polymérase Bst, l'ADN polymérase LA Taq et rTth.

9. Procédé selon la revendication 1, dans lequel l'étape d'extension comprend un dNTP marqué par la digoxigénine qui est incorporé dans les produits d'extension.

10. Procédé selon la revendication 1, dans lequel la première amorce est étendue d'au moins environ 10 000 bases.

11. Procédé selon la revendication 1, dans lequel la première amorce est étendue d'au moins environ 100 000 bases.

12. Procédé selon la revendication 1, dans lequel ladite première amorce est étendue sur une région comprenant entre 50 et 1 000 polymorphismes.

13. Procédé selon la revendication 1, dans lequel ladite pluralité de sondes sont attachées à un support solide.

14. Procédé selon la revendication 1, dans lequel ladite pluralité de sondes sont des sondes oligonucléotidiques qui ont une longueur comprise entre 20 et 80 bases et dans lequel ledit réseau comprend au moins 500 000 sondes différentes qui sont présentes à des emplacements connus ou déterminables.

15. Procédé selon la revendication 6, comprenant en outre une purification par affinité des produits d'extension d'amorce, qui comprend une incubation des produits d'extension d'amorce avec un anticorps anti-biotine conjugué à de l'agarose afin de lier les produits d'extension d'amorce à l'agarose, et une élimination de l'acide nucléique non lié.

16. Procédé selon la revendication 8, dans lequel ladite polymérase est l'ADN polymérase phi29.

17. Procédé selon la revendication 1, dans lequel ladite première amorce est résistante à une digestion par une exonucléase 5' à 3' et ledit procédé comprenant en outre la digestion des produits d'extension d'amorce générés avec une exonucléase 5' à 3'.

18. Procédé selon la revendication 8, dans lequel ladite ADN polymérase est l'ADN polymérase Bst.
